# EUROPÄISCHE PATENTANMELDUNG

(11) **EP 0 553 615 A1**
(43) Veröffentlichungstag der Anmeldung: **04.08.1993**
(21) Anmeldenummer: 93100137.4
(22) Anmeldetag: 07.01.1993
(51) Int. Cl.: A61F 13/08

(54) **Kosmetische Kompressionsvorrichtung**

(30) Priorität: 29.01.1992 DE 9201000 U
(71) Anmelder: Weyergans, Rudolf, D-52064 Aachen (DE)
(72) Erfinder: Weyergans, Rudolf, D-52064 Aachen (DE)

(57) **Zusammenfassung**

Die Erfindung bezeichnet eine preiswerte und leicht handhabbare Möglichkeit, im Fall von kosmetisch relevanter Orangenhaut den Abbau zurückbehaltener großmolekularer Stoffwechsel-Restprodukte aufgrund einer Verbesserung der Ausbeute der physiologischen Muskelpumpe zu steigern. Sie besteht in einer Kompressionsvorrichtung aus mehreren Kompressionsstrümpfen oder Kompressionsstrumpfhosen oder Bandagen, die standardmäßig produziert werden können, und übereinander angelegt werden. Dabei können zur Vereinfachung und zur Sicherung des richtigen Umgangs mit dem Produkt die einzelnen Teile fest oder variabel miteinander verbunden werden.

## Beschreibung

Die Erfindung bezieht sich auf die Kompressionsbehandlung von menschlichen Extremitäten zur Verbesserung der Effizienz der physiologischen Muskelpumpe im kosmetischen Bereich.

Neben dem medizinischen Bereich, in dem Kompressionstherapien bei venöser Erkrankungen eingesetzt werden, besteht auch im nicht-medizinischen resp. kosmetischen Bereich das Bedürfnis, Kompressionsvorrichtungen einzusetzen; dabei ist es das Ziel der Kompressionstherapie hier, Einfluß auf den Abbau der sogenannten Cellulite oder 'Orangenhaut' auszuüben.

Bei Cellulite resp. Orangenhaut handelt es sich um großmolekulare Ablagerungen von festen Stoffwechsel-Restprodukten in den Zellen des Unterhaut-Fettgewebes vornehmlich an Oberschenkel, Hüfte und Armen. Derartige feste, großmolekulare Stoffwechsel-Restprodukte sind lymphpflichtig; d.h. sie müssen über das lymphatische System des Organismus abdrainiert werden.

Antrieb des lymphatischen Systems ist mangels eines eigenes Herzens die sogenannte physiologische Muskelpumpe; d.h. Lymphflüssigkeit fließt nur dann ausreichend, wenn man sich bewegt, - also dann, wenn die beteiligten Muskelgruppen des jeweiligen Körperareals ausreichend angespannt und entspannt werden. Dabei pumpen die Muskeln zusammen mit der Lymphflüssigkeit auch die lymphpflichtigen Stoffwechsel-Restprodukte aus den Extremitäten heraus in den Rumpf und zu den Filterorganen.

Die physiologische Muskelpumpe funktioniert aber nur dann, wenn die Haut aus kosmetischer Sicht 'straff' ist, - d.h. wenn das Bindegewebe der Haut entsprechenden Gegendruck ausübt. In dem Fall, daß das Bindegewbe z.B. alterungsbedingt erschlafft ist, verpuffen Teile des Muskelpumpendrucks durch die oberen Hautschichten in die Atmosphäre. Das bewirkt, daß Lymphe nur unzureichend fließt und daß dementsprechend Stoffwechsel-Restprodukte in den Extremitäten zurückbehalten werden. Die Fettzellen blähen sich auf; diese Situation wird als 'Orangenhaut' bezeichnet.

Dieser Situation kann durch eine Kompressionsvorrichtung abgeholfen werden, die die Funktion eines straffen, intakten Bindegewebes imitiert, und bewirkt, daß die Muskelpumpe effizient ausgenutzt wird.

Herkömmliche Kompressionsbandagierungen und Kompressionsstrümpfe im medizinischen Bereich sind jedoch aufwendig und teuer; medizinische Kompressionsstrumpfhosen werden sogar individuell maßgeschneidert und das Anlegen ist ebenfalls sehr aufwendig und dauert mehrere Minuten.

Die Erfindung benennt eine kosmetische Kompressionsvorrichtung, die einfach und kostengünstig zu produzieren und leicht handhabbar ist, und die dennoch ausreichenden Kompressionsdruck für die besonderen Belange der kosmetischen Behandlung der Orangenhaut ausübt.

Die Erfindung sieht vor, zwei oder mehrere Kompressionsstrümpfe bzw. Kompressionsstrumpfhosen übereinander anzuziehen.

Jeder der Kompressionsstrümpfe oder Strumpfhosen hat für sich alleine nur eine geringe Kompressionswirkung und läßt sich mit Leichtigkeit und schnell anlegen. Dadurch, daß zwei oder mehr Strümpfe und/oder Strumpfhosen übereinander angezogen werden, erhöht sich der Druck auf die Haut, bis ein ausreichender Gegendruck auf die Muskelpumpe entsteht.

Um die richtige Verwendung zu sichern, können die beiden oder mehrere Strümpfe (Unter- und Oberstrumpf) an einem Ende (z.B. an den Zehenspitzen) oder an einer anderen Stelle miteinander verbunden werden.

Die Verbindung von Unter- und Oberstrümpfen kann auch variabel gestaltet werden, etwa durch Druckknöpfe, Klettverschlüsse, Bänder o.ä..

Gegenstand der Erfindung ist auch, mit Hilfe partieller Kompressionsbandagen den Kompressionsdruck an bestimmten Stellen zu erhöhen; hierbei ist es gleich, ob die partiellen Bandagen fixiert werden oder unfixiert bleiben.

## Patentansprüche

1. Kosmetische Kompressionsvorrichtung, die die Füße, Beine, Gesäß und/oder Hüfte oder Arme ganz oder teilweise enganliegend bedeckt, dadurch gekennzeichnet, daß sie aus mindestens zwei einander bedeckenden gleichen oder ähnlichen Einzelkompressionsstrümpfen oder Einzelkompressionsstrumpfhosen besteht.

2. Kosmetische Kompressionsvorrichtung nach Anspruch 1, dadurch gekennzeichnet, daß die einander bedeckenden Einzelkompressionsstrümpfe bzw. -strumpfhosen an mindestens einer Stelle miteinander verbunden sind.

3. Kosmetische Kompressionsvorrichtung nach Anspruch 2, dadurch gekennzeichnet, daß die Einzelkompressionsstrümpfe bzw. -strumpfhosen durch Druckknöpfe, Knöpfe, Klettband oder dgl. lösbar miteinander verbindbart sind.

4. Kosmetische Kompressionsvorrichtung nach einem der Ansprüche 1 bis 3, dadurch gekennzeichnet, daß nur Teile von Einzelkompressionsstrümpfen bzw. -strumpfhosen eine oder mehr übereinanderliegende Einzelstrümpfe oder Einzelstrumpfhosen bedecken.
